# EUROPEAN PATENT APPLICATION

(11) **EP 3 360 894 A1**
(43) Date of publication of application: **15.08.2018**
(21) Application number: 16864438.3
(22) Date of filing: 13.07.2016
(51) Int. Cl.: C07K 14/705, C07K 16/28, A61K 8/64, A61K 39/395, A61Q 9/04

(54) **FUNCTIONAL REGULATORY ANTIBODY HAVING ACTIONS OF PROMOTING PROLIFERATION, SURVIVAL, AND CELL ACTIVATION OF DERMAL PAPILLA CELLS AS HAIR GROWTH REGULATING CENTER CELLS AND USE THEREOF**

(30) Priority: 12.11.2015 KR 20150158897
(71) Applicant: Kim, Dong Ku, Seoul 01603 (KR)
(72) Inventor: Kim, Dong Ku, Seoul 01603 (KR)
(74) Representative: Brevalex
(86) International application number: PCT/KR2016/007598
(87) International publication number: WO 2017/082512

(57) **Abstract**

The present invention relates to a functional antibody, which specifically binds to only dermal papilla cells and dermal sheath cells and has efficacies of proliferation, survival, and activation of cells, and to a use thereof and, more specifically, to a pharmaceutical composition/cosmetic composition for preventing hair loss or promoting hair growth using the functional antibody, and to a method for preventing hair loss or promoting hair growth. It was verified that the functional antibody promotes the proliferation, survival, and activation of dermal papilla cells through cell signaling by binding with a receptor protein specifically expressed in only dermal papilla cells, thereby promoting hair growth. Therefore, the present invention can perform target therapy through a more fundamental approach in the prevention or treatment of hair loss, and is expected to also contribute to the production of cell therapeutic agents containing dermal papilla cells.

## Description

### TECHNICAL FIELD

The present disclosure relates to an antibody which plays a role in promoting proliferation, viability, and activation of dermal papilla cells, and use of the antibody, and more specifically, to a pharmaceutical composition/cosmetic composition for preventing hair loss or promoting hair growth using the antibody, and a method of preventing hair loss or promoting hair growth.

### BACKGROUND ART

Hair loss refers to a state in which hairs are not present at a site where hairs should be normally present, and generally, means loss of terminal hairs (coarse dark hairs) of the scalp. The number of hairs on a human is about 100,000 to about 150,000, and hair has a unique hair growth cycle of anagen, catagen, and telogen phases, and repeats growth and loss. On average, about 50 to 70 hairs a day are lost naturally, and a mechanism for hair loss has not yet been clearly elucidated. Studies that have been conducted so far reported that hair loss is likely to be caused by excessive sebum production due to disorders of the endocrine system such as hormonal imbalances, etc., abnormalities of the circulatory system such as autonomic nervous system and blood circulation disorders, etc., malnutrition of the hair follicles, allergies, bacterial infections, genetic factors, psychological stress, environmental factors such as air pollution or food contamination, aging, etc.

Meanwhile, the number of people with hair loss in Korea is about 10 million, meaning that one in five people suffers from hair loss. Generally, hair loss occurs mainly in middle-aged people. Recently, however, hair loss has also been occurring frequently in young people in their 20s and 30s, and the age of hair loss patients is gradually decreasing. According to the Health Insurance Review and Assessment Service, the number of people under treatment for hair loss in Korea is increasing by 3.6% every year. In 2013, the percentage of those in their 20s and 30s with hair loss reached 45%, with women accounting for more than 40% of this percentage, indicating that people of various ages are suffering from hair loss.

Such hair loss lowers quality of life due to psychological stress, and interferes with interpersonal relationships and social life. In a person's social life, appearance gives an impression, and physical appearance becomes an invisible influence on their self-esteem and social life. In this sense, there is a growing interest in the causes and treatments of hair loss.

Accordingly, many studies have been conducted to alleviate or treat hair loss, and in particular, over a long period, research has been conducted in order to develop new materials for stimulating hair growth or alleviating hair loss in the cosmetics or pharmaceutical industries. As a result, drug therapies using female hormones, blood flow promoters, etc., and hair transplantation methods have been developed. The most commonly used drugs for hair loss treatment are 2,4-diamino-6-piperidinopyrimidine-3-oxide (also called 'Minoxidil' formulation, see US Patent Nos. 4,139,619 and 4,596,812) and finasteride, which is a specific inhibitor of type II 5α reductase, and these two drugs are approved by the FDA.

A formulation called minoxidil was developed as a therapeutic agent for hypertension by increasing blood flow through vasodilatory effects, but these effects can also induce hair growth by expanding the blood vessels connected to the hair follicles which receive various nutrients from the blood. Minoxidil, which is commercially available under the name of ROGAINE (the registered trademark of Pharmacia & Upjohn Company), is mainly used in female and male hair loss patients. However, minoxidil used for external application should be regularly used for a long period of 6 months or longer, and its hair growth effect is very small. Further, minoxidil is effective against hair loss rather than hair regeneration, and clinical studies have shown that it is effective only for 30% or less of patients with hair loss. A hair loss treatment composition including minoxidil is problematic in that it is inconvenient to use because of its odor and high viscosity, and produces hair discharge such that hair sticks together, which further causes skin irritation due to unsanitary problems occurring in the scalp.

Further, a drug containing finasteride as an active ingredient is commercially available under the trade name of PROPECIA (the registered trademark of Merck & Co., Inc.), and was originally developed as a therapeutic agent for the prostate, but it has been used not only as a prostate treatment agent but also as a hair growth agent, after its effect on hair growth was revealed in clinical trials. This drug is a pill for oral administration, which prevents conversion of the male hormone testosterone to dihydrotestosterone (DHT) by inhibiting the function of type II 5α reductase, and does not directly affect hair regeneration. This drug must also be taken continuously and regularly, and male patients with hair loss have side effects such as loss of sexual desire, erectile dysfunction, etc. due to male hormone suppression, and prescription of the drug for women and children is prohibited.

Accordingly, in order to solve the above problems, many studies have been actively conducted on new hair loss treatment agents (Korean Patent Publication NO. 10-2015-0083054), but the outcome is still unsatisfactory.

### DESCRIPTION OF EMBODIMENTS

### TECHNICAL PROBLEM

The present disclosure has been made in order to solve the above problems. In the process of hair regeneration, dermal papilla cells secrete proteins that regulate proliferation and differentiation of hair follicle stem/progenitor cells and play a pivotal role in regulating the hair growth cycle. The present inventors first identified a functional antibody protein that specifically binds to the receptor protein IGDCC4 which is expressed only in cell membranes of dermal papilla cells, thereby inducing proliferation and activation of dermal papilla cells and promoting production of various regulatory factors involved in hair regeneration, leading to the present disclosure.

Accordingly, an object of the present disclosure is to provide an IGDCC4 antigen protein having an amino acid sequence represented by SEQ ID NO: 17.

Further, another object of the present disclosure is to provide an anti-IGDCC4 functional antibody or a fragment thereof including an antigen-binding site, in which the anti-IGDCC4 functional antibody induces proliferation, survival, and activation of dermal papilla cells via specific binding with the IGDCC4 antigen protein.

Further, still another object of the present disclosure is to provide a method of preparing the anti-IGDCC4 antibody, the method including preparing a functional antibody protein which directly binds to the IGDCC4 antigen protein to promote functions of dermal papilla cells.

Further, still another object of the present disclosure is to provide an expression vector for producing the anti-IGDCC4 antibody or the fragment thereof, wherein the anti-IGDCC4 antibody includes isolated polynucleotides encoding amino acid sequences having SEQ ID NO: 7 and SEQ ID NO: 8; a host cell transformed with the expression vector; and a method of producing the anti-IGDCC4 antibody by using the host cell *in vitro.*

Further, still another object of the present disclosure is to provide a pharmaceutical composition and/or a cosmetic composition for preventing hair loss or promoting hair growth, the composition including any one selected from the group consisting of a ligand, an antibody protein, a polypeptide, and a small molecule, each of which specifically binds to the IGDCC4 antigen protein.

Further, still another object of the present disclosure is to provide a method of preventing hair loss or promoting hair growth, the method including applying or spraying the composition onto the scalp.

Further, still another object of the present disclosure is to provide a method of producing a large amount of dermal papilla cells and dermal sheath cells, the method including a) isolating dermal papilla cells or dermal sheath cells from the scalp; and b) treating the isolated dermal papilla cells or dermal sheath cells with the anti-IGDCC4 antibody.

Further, still another object of the present disclosure is to provide a cell therapeutic agent for hair regeneration, the cell therapeutic agent including the dermal papilla cells and the dermal sheath cells produced by the above method.

Further, still another object of the present disclosure is to provide use of any one selected from the group consisting of a ligand, an antibody protein, a polypeptide, and a small molecule, each of which specifically binds to the IGDCC4 antigen protein, in the preparation of the pharmaceutical or cosmetic composition for preventing hair loss or promoting hair growth.

Further, still another object of the present disclosure is to provide a method of treating hair loss, the method including administering the pharmaceutical composition to a subject, wherein the pharmaceutical composition includes as an active ingredient a pharmaceutically effective amount of any one selected from the group consisting of a ligand, an antibody protein, a polypeptide, and a small molecule, each of which specifically binds to the IGDCC4 antigen protein.

It is to be understood, however, that the technical problems of the present disclosure are not limited to the above-mentioned objects, and other problems not mentioned may be clearly understood by those skilled in the art from the following description.

### SOLUTION TO PROBLEM

In order to achieve the above objects of the present disclosure, the present disclosure provides an IGDCC4 antigen protein having an amino acid sequence represented by SEQ ID NO: 17.

Further, the present disclosure provides an anti-IGDCC4 functional antibody or a fragment thereof including an antigen-binding site, in which the anti-IGDCC4 functional antibody induces proliferation, survival, and activation of dermal papilla cells via specific binding with the IGDCC4 antigen protein.

In one specific embodiment of the present disclosure, the antibody may include a heavy chain variable region including one or more selected from the group consisting of HCDR1 having an amino acid sequence represented by SEQ ID NO: 1, HCDR2 having an amino acid sequence represented by SEQ ID NO: 2, and HCDR3 having an amino acid sequence represented by SEQ ID NO: 3; and a light chain variable region including one or more selected from the group consisting of LCDR1 having an amino acid sequence represented by SEQ ID NO: 4, LCDR2 having an amino acid sequence represented by SEQ ID NO: 5, and LCDR3 having an amino acid sequence represented by SEQ ID NO: 6.

In another specific embodiment of the present disclosure, the antibody may include a heavy chain variable region having an amino acid sequence represented by SEQ ID NO: 7 and a light chain variable region having an amino acid sequence represented by SEQ ID NO: 8.

In still another specific embodiment of the present disclosure, the antibody may include a heavy chain variable region including one or more selected from the group consisting of HCDR1 having a polynucleotide sequence represented by SEQ ID NO: 9, HCDR2 having a polynucleotide sequence represented by SEQ ID NO: 10, and HCDR3 having a polynucleotide sequence represented by SEQ ID NO: 11; and a light chain variable region including one or more selected from the group consisting of LCDR1 having a polynucleotide sequence represented by SEQ ID NO: 12, LCDR2 having a polynucleotide sequence represented by SEQ ID NO: 13, and LCDR3 having a polynucleotide sequence represented by SEQ ID NO: 14.

In still another specific embodiment of the present disclosure, the antibody may include a heavy chain variable region having a polynucleotide sequence represented by SEQ ID NO: 15 and a light chain variable region having a polynucleotide sequence represented by SEQ ID NO: 16.

In still another specific embodiment of the present disclosure, the antibody may be a monoclonal antibody or a polyclonal antibody.

In still another specific embodiment of the present disclosure, the antibody may include any one light chain or heavy chain variable region selected from the group consisting of Fab, Fab', F(ab')₂, dsFv, scFv, sc(Fv)₂, IgNAR, hcIgG, (scFv-SA)₄, [sc(Fv)₂]₂, minibody, diabody, tribody, nanobody, and camelbody.

Further, the present disclosure provides a method of preparing the anti-IGDCC4 antibody, the method including injecting the IGDCC4 antigen protein into an animal.

Further, the present disclosure provides an expression vector for producing the anti-IGDCC4 antibody or the fragment thereof, wherein the anti-IGDCC4 antibody includes isolated polynucleotides encoding amino acid sequences having SEQ ID NO: 7 and SEQ ID NO: 8; a host cell transformed with the expression vector; and a method of producing the anti-IGDCC4 antibody by using the host cells *in vitro.*

In one specific embodiment of the present disclosure, the host cell may be any one selected from the group consisting of microorganisms such as bacteria (*E. coli*)*,* yeast, etc., CHO cells, F2N cells, HEK293 cells, and antibody-producing hybridoma cells.

Further, the present disclosure provides a pharmaceutical/cosmetic composition for preventing hair loss or promoting hair growth, the composition including any one selected from the group consisting of a ligand, an antibody protein, a polypeptide, and a small molecule, each of which specifically binds to the IGDCC4 antigen protein.

In one specific embodiment of the present disclosure, the composition may promote proliferation, survival, and activation of the dermal papilla cells by specifically binding to dermal papilla cells or dermal sheath cells.

In another specific embodiment of the present disclosure, the pharmaceutical composition may be prepared into a formulation selected from the group consisting of a cream, a gel, a patch, a spray, an ointment, a plaster, a lotion, a liniment, a paste, and a cataplasma.

In still another specific embodiment of the present disclosure, the cosmetic composition may be prepared into a formulation selected from the group consisting of a hair tonic, a hair conditioner, a hair essence, a hair lotion, a hair-nourishing lotion, a hair shampoo, a hair rinse, a hair treatment, a hair cream, a hair-nourishing cream, a hair moisturizing cream, a hair massage cream, a hair wax, a hair aerosol, a hair pack, a hair-nourishing pack, a hair soap, a hair cleansing foam, a hair oil, a hair drying preparation, a hair preserving and managing preparation, a hair dye, a hair waving preparation, a hair bleaching agent, a hair gel, a hair glaze, a hair dressinger, a hair lacquer, a hair moisturizer, a hair mousse, and a hair spray.

Further, the present disclosure provides a method of preventing hair loss or promoting hair growth, the method including applying or spraying the cosmetic composition onto the scalp.

Further, the present disclosure provides a method of producing a large amount of dermal papilla cells and dermal sheath cells, the method including a) isolating dermal papilla cells or dermal sheath cells from the scalp; and b) treating the isolated dermal papilla cells or dermal sheath cells with the anti-IGDCC4 antibody.

Further, the present disclosure provides a cell therapeutic agent for hair regeneration, the cell therapeutic agent including the dermal papilla cells and the dermal sheath cells produced by the above method.

Further, the present disclosure provides use of the anti-IGDCC4 antibody or the fragment thereof including an antigen-binding site in preventing hair loss or promoting hair growth.

Further, the present disclosure provides use of any one selected from the group consisting of a ligand, an antibody protein, a polypeptide, and a small molecule, each of which specifically binds to the IGDCC4 antigen protein in the preparation of the pharmaceutical or cosmetic composition for preventing hair loss or promoting hair growth.

Further, the present disclosure provides a method of treating hair loss, the method including administering the pharmaceutical composition to a subject, wherein the pharmaceutical composition includes as an active ingredient a pharmaceutically effective amount of any one selected from the group consisting of a ligand, an antibody protein, a polypeptide, and a small molecule, each of which specifically binds to the IGDCC4 antigen protein.

### ADVANTAGEOUS EFFECTS OF DISCLOSURE

The present disclosure relates to an antibody specifically binding to dermal papilla cells and use thereof. It was confirmed that the antibody specifically binds to dermal papilla cells to promote proliferation of dermal papilla cells, thereby promoting hair growth.

Further, the present disclosure relates to a therapeutic agent for hair loss using a protein formulation, in which the therapeutic agent solves problems of ROGAINE which is currently used as a therapeutic agent for hair loss, such as long-term use and low clinical efficacy, and side effects of PROPECIA, such as loss of libido, and has advantages of very excellent binding specificity and *in vivo* stability, which are features of antibody proteins. The antibody of the present disclosure specifically binds to dermal papilla cells to promote proliferation, survival, and activation of dermal papilla cells, thereby preventing hair loss. Accordingly, it is expected that the antibody of the present disclosure may be used as a fundamental therapeutic agent for hair loss patients at the telogen phase.

It is also expected that the antibody of the present disclosure may be used as a cell therapeutic agent for hair regeneration through *in vitro* mass-production of dermal papilla cells or dermal sheath cells by using the antibody protein of the present disclosure and direct transplantation of the cells.

### BRIEF DESCRIPTION OF DRAWINGS

FIG. 1 shows results of flow cytometry performed to examine binding of anti-IGDCC4 antibody protein (HARE57) and human dermal papilla cells;
FIG. 2 shows a result of fluorescent microscopy used to examine binding of anti-IGDCC4 antibody protein (HARE57) and human dermal papilla cells;
FIG. 3 shows results of examining binding of anti-IGDCC4 antibody protein, dermal papilla cells, and dermal sheath cells in the skin tissue of a mouse, in which (A) represents the result of optical microscopy and (B) represents the result of fluorescent microscopy;
FIG. 4 shows results of optical and fluorescent microscopy performed to examine binding of anti-IGDCC4 antibody protein in brain, heart, kidney, liver, and lung tissues, in which (A) represents the binding in control groups, and (B) represents the binding in anti-IGDCC4 antibody protein (HARE57)-treated groups;
FIG.5 shows results of flow cytometry performed to examine binding of anti-IGDCC4 antibody protein and human blood cells, in which (A) represents the binding in control groups, and (B) represents the binding in anti-IGDCC4 antibody protein (HARE57)-treated groups;
FIG. 6 shows changes in proliferation rates of human dermal papilla cells according to treatment with anti-IGDCC4 antibody protein;
FIG. 7 shows morphological changes of human dermal papilla cells due to treatment with anti-IGDCC4 antibody protein, in which (A) represents the morphological change in a control group, and (B) represents the morphological change in an anti-IGDCC4 antibody protein-treated groups;
FIG. 8 shows result of examining hair growth promotion with the naked eye in 6-week-old C3H mice which were not treated with anti-IGDCC4 antibody protein;
FIG. 9 shows result of examining hair growth promotion with the naked eye in 6-week-old C3H mice which were treated with anti-IGDCC4 antibody protein;
FIG. 10 shows result of examining hair growth promotion with the naked eye in nude mice (BALB/c-nude) which were treated with anti-IGDCC4 antibody protein;
FIG. 11 shows results of examining hair growth promoting effects in stress-induced hair loss mice by treatment of anti-IGDCC4 antibody protein, wherein (A) and (B) shows results of examining hair growth promotion with the naked eye;
FIG. 12 shows a result of comparing IGDCC4 mRNA expression in dermal papilla cells and HeLa cells according to treatment with anti-IGDCC4 antibody protein;
FIG. 13 shows a result of ELISA for examining binding of anti-IGDCC4 antibody protein (HARE57) with IGDCC4 protein;
FIG. 14 shows results of protein array analysis for examining protein expression patterns in dermal papilla cells according to treatment with anti-IGDCC4 antibody protein (HARE57);
FIG. 15 shows results of examining representative hair growth-regulating factors (ALPP, cytokeratin19, EDA-A2, FGF-5, FGF-6, FGFR1, FGFR2, IL-1α, and IL-6) of which expression was increased by treatment of dermal papilla cells with anti-IGDCC4 antibody protein;
FIG. 16 shows results of examining proteins of which expression was increased by treatment of dermal papilla cells with anti-IGDCC4 antibody protein, in which (A) represents increased expression of the FGF family, (B) represents increased expression of the Wnt family, (C) represents increased expression of the TGF family, (D) represents increased expression of the PDGF family, (E) represents increased expression of the ECM family, and (F) represents increased expression of the TNF family;
FIG. 17 shows results of examining cell proliferation-regulating proteins of which expression was increased by treatment of dermal papilla cells with anti-IGDCC4 antibody protein; and
FIG. 18 shows results of examining blood cell-regulating factors of which expression was increased by treatment of dermal papilla cells with anti-IGDCC4 antibody protein.

### BEST MODE

The present inventors demonstrated that an antibody of the present disclosure specifically binds to only dermal papilla cells to promote proliferation, survival, and activation of the dermal papilla cells. Based on this result, they found that treatment of the antibody may increase hair growth effects and expression levels of hair growth-related regulatory factors, thereby completing the present disclosure.

Hereinafter, the present disclosure will be described in detail.

The present disclosure provides an IGDCC4 antigen protein having an amino acid sequence represented by SEQ ID NO: 17.

In the present disclosure, the IGDCC4 antigen protein is not limited, as long as it is derived from a mammal, and preferably, the IGDCC4 antigen protein may be derived from humans, mice, or rats. In this regard, the IGDCC4 antigen protein may include a nucleotide sequence having 70% or more, preferably 80% or more, more preferably 90% or more, most preferably 95% or more sequence homology with the amino acid sequence represented by SEQ ID NO: 17.

The present disclosure provides a method of producing the anti-IGDCC4 antibody, the method including injecting the IGDCC4 antigen protein into an animal.

Further, the present disclosure provides an anti-IGDCC4 antibody specifically binding to the IGDCC4 antigen protein, a fragment thereof including an antigen-binding site, or a functional variant thereof.

As used herein, the term "antibody" includes an immunoglobulin molecule which is immunologically reactive to a specific antigen, and includes all of a polyclonal antibody and a monoclonal antibody. Further, the term "antibody" may include an antibody, an antibody fragment, a chimeric antibody (e.g., humanized murine antibody) and a bivalent antibody (e.g., bispecific antibody) produced by genetic engineering.

Generally, an antibody has a heavy chain and a light chain, and each of the heavy chain and the light chain includes a constant region and a variable region (the region is also known as "domain"). The variable regions of the light chain and the heavy chain include three hypervariable regions called complementarity-determining region (hereinafter, "CDR") and four "framework regions". The CDR mostly plays a role in binding to an epitope of an antigen. The CDR of each chain is generally called sequentially starting from the N-terminus, CDR1, CDR2, and CDR3, and is identified by a chain to which a particular CDR is located.

In the present disclosure, the CDR may include Fab, Fab', F(ab')₂, dsFv, scFv, sc(Fv)₂, IgNAR, hcIgG, (scFv-SA)₄, [sc(Fv)₂]₂, minibody, diabody, tribody, nanobody, or camelbody, but is not limited thereto.

In the present disclosure, CDR1 (HCDR1), CDR2 (HCDR2), CDR3 (HCDR3) of the heavy chain variable region may have amino acid sequences represented by SEQ ID NOS: 1 to 3, respectively and CDR1 (LCDR1), CDR2 (LCDR2), CDR3 (LCDR3) of the light chain variable region may have amino acid sequences represented by SEQ ID NOS: 4 to 6, respectively. They may have include nucleotide sequences having 70% or more, preferably 80% or more, more preferably 90% or more, most preferably 95% or more sequence homology with the amino acid sequences represented by SEQ ID NOS: 1 to 6, respectively.

CDR1 (HCDR1), CDR2 (HCDR2), CDR3 (HCDR3) of the heavy chain variable region may have polynucleotide sequences represented by SEQ ID NOS: 9 to 11, respectively and CDR1 (LCDR1), CDR2 (LCDR2), CDR3 (LCDR3) of the light chain variable region may have polynucleotide sequences represented by SEQ ID NOS: 12 to 14, respectively. They may have include nucleotide sequences having 70% or more, preferably 80% or more, more preferably 90% or more, most preferably 95% or more sequence homology with the polynucleotide sequences represented by SEQ ID NOS: 9 to 14, respectively.

Further, the heavy chain variable region may have an amino acid sequence represented by SEQ ID NO: 7 and the light chain variable region may have an amino acid sequence represented by SEQ ID NO: 8, and they may have include nucleotide sequences having 70% or more, preferably 80% or more, more preferably 90% or more, most preferably 95% or more sequence homology with the amino acid sequences represented by SEQ ID NOS: 7 and 8, respectively.

Further, the heavy chain variable region may have a polynucleotide represented by SEQ ID NO: 15 and the light chain variable region may have a polynucleotide represented by SEQ ID NO: 16, and they may have include nucleotide sequences having 70% or more, preferably 80% or more, more preferably 90% or more, most preferably 95% or more sequence homology with the polynucleotide sequences represented by SEQ ID NOS: 15 and 16, respectively.

Further, the functional variants of the anti-IGDCC4 antibody include biological equivalents of the anti-IGDCC4 antibody sequences described herein, as long as the functional variants are able to specifically recognize IGDCC4. For example, in order to further improve binding affinity and/or other biological characteristics of the antibody, the amino acid or polynucleotide sequence thereof may be additionally altered. Such alterations include, for example, deletion, insertion, and/or substitution of amino acid residues of the antibody, and the alterations are based on the relative similarity of the amino acid side-chain substituents, for example, their hydrophobicity, hydrophilicity, charge, size, etc. An analysis of the size, shape, and type of the amino acid side-chain substituents may reveal that arginine, lysine, and histidine are all positively charged residues; alanine, glycine, and serine are similar in size; and phenylalanine, tryptophan, and tyrosine are similar in shape. Therefore, on the basis of these considerations, arginine, lysine, and histidine; alanine, glycine, and serine; and phenylalanine, tryptophan, and tyrosine may be considered to be biologically functional equivalents.

Further, the present disclosure provides an expression vector for producing the anti-IGDCC4 antibody or the fragment thereof, wherein the anti-IGDCC4 antibody includes isolated polynucleotides encoding the amino acid sequences having SEQ ID NO: 7 and SEQ ID NO: 8; a host cell transformed with the expression vector; and a method of producing the anti-IGDCC4 antibody by using the host cells *in vitro.*

As used herein, the "transformation" means a molecular biological technique that changes the genetic trait of a cell by a DNA chain fragment or plasmid that possess a different type of foreign gene from that of the original cell, penetrates among the cells, and combines with DNA that existed in the original cell. With respect to the objects of the present disclosure, the transformation means that the isolated polynucleotides encoding the amino acid sequences represented by SEQ ID NO: 7 and SEQ ID NO: 8 are inserted into the host cells to produce the anti-IGDCC4 antibody or the fragment thereof.

The host cell may be preferably any one selected from the group consisting of microorganisms such as bacteria (E. *coli*)*,* yeast, etc., CHO cells, F2N cells, HEK293 cells, and antibody-producing hybridoma cells, but are not limited thereto.

In one embodiment of the present disclosure, an antibody protein (HARE57 antibody protein) binding to human dermal papilla cells was prepared, and it was confirmed that binding of the antibody protein and the skin, brain, heart, kidney, liver, and lung tissues of a mouse animal model or human blood cells was not observed, whereas binding of the antibody protein and dermal papilla cells or dermal sheath cells was observed, indicating binding specificity of the antibody protein for dermal papilla cells (see Examples 1 and 2). It was also confirmed that proliferation, survival, and activation of the dermal papilla cells and *in vivo* hair growth effect were promoted by treatment of the antibody protein. IGDCC4 which is a cell membrane protein of the dermal papilla cells and specifically binds with the antibody was identified, and the anti-IGDCC4 antibody (HARE57 antibody protein) binds with IGDCC4 expressed in dermal papilla cells, thereby increasing expression levels of hair growth-related regulatory factors which are promoted via cell signaling pathways, indicating that the antibody of the present disclosure may be used for preventing hair loss or promoting hair growth (see Examples 3 to 5).

Accordingly, the present disclosure provides a pharmaceutical composition for preventing hair loss or promoting hair growth, the composition including the antibody or the fragment thereof including an antigen-binding site as an active ingredient.

As used herein, the term "hair loss" refers to a state in which hairs are not present at a site where hairs should be normally present, and generally, means loss of terminal hairs (coarse dark hair) of the scalp. Contrary to previous perceptions, hair loss is not limited to men but is also greatly increasing in women, and teenagers worrying about hair loss are also increasing. There is a growing interest in therapeutic agents effective for hair loss.

There are two kinds of hair loss therapeutic agents, minoxidil (transdermal application) and finasteride (oral preparation) approved by FDA. First, minoxidil was used as an oral antihypertensive drug, but hirsutism was observed in patients taking this drug. Thus, minoxidil is currently used for male hair loss by topical application onto the scalp. Further, finasteride is used as an oral therapeutic agent for male androgenic hair loss by inhibiting type II 5α-reductase to prevent hair loss and to promote hair growth.

However, many clinical studies reported that minoxidil is effective only 30% or less of patients, and when stop using minoxidil, hair loss progresses, and side effects such as weight gain, swelling, rapid heartbeat, angina, dermatitis, itching, etc. occur. Further, finasteride has serious side effects such as male sexual dysfunction, birth of deformed child, etc., which are considered to be clinical cases, and therefore, its use is limited or patients themselves are reluctant to use finasteride. Accordingly, consumers' interest in hair loss materials using protein preparations with high *in vivo* stability is increasing.

In general, hair growth occurs by a complex mechanism caused by various genes, growth factors and their receptors, and systemic actions of hormones. In particular, follicular dermal matrix cells composed of epithelial cells and dermal papilla cells composed of mesenchymal cells are known to act as a pivotal factor in the formation and growth of hair.

Dermal papilla cells are cells that are located at the base of the hair follicles, and supply oxygen and nutrients to the cells that constitute the hair follicles, and regulates hair growth and follicle cycle. Further, since hair growth progresses as epithelial cells surrounding the dermal papilla divide and form a hair shaft, dermal papilla cells play a role in regulating division of epithelial cells. Considering that male hormone acts on dermal papilla cells in hair follicle in male hair loss, promotion of the proliferation of dermal papilla cells may make hair healthier, promote hair growth, and prevent hair loss. Further, dermal sheath cells are cells that have ability to reversibly differentiate to dermal papilla cells, and play an important role in hair growth, like dermal papilla cells.

Based on this theory, the present inventors suggest an alternative to overcome the problems of known hair loss therapeutic agents, and provide a pharmaceutical/cosmetic composition for preventing hair loss, the composition including any one selected from the group consisting of a ligand, an antibody protein, a polypeptide, and a small molecule, of which specifically binds to the IGDCC4 antigen protein of Claim 1.

The composition specifically binds to dermal papilla cells or dermal sheath cells to promote proliferation and survival of the dermal papilla cells and to activate the dermal papilla cells, thereby preventing hair loss and promoting hair growth, and preferably, the composition may include the antibody protein specifically binding to the IGDCC4 antigen protein, but the composition may include any one without limitation, as long as it binds to the IGDCC4 antigen protein to regulate the functions of dermal papilla cells.

It is intended to provide a pharmaceutical composition, which specifically binds to dermal papilla cells or dermal sheath cells to promote proliferation and survival of the dermal papilla cells and to activate the dermal papilla cells, thereby preventing hair loss and promoting hair growth.

The pharmaceutical composition of the present disclosure may have any formulation for parenteral administration, selected from the group consisting of a cream, a gel, a patch, a spray, an ointment, a plaster, a lotion, a liniment, a paste, and a cataplasma upon clinical administration, but is not limited thereto.

For formulation into a parenteral dosage form, a stabilizer or a buffer may be mixed with water to prepare a solution or suspension, which may be prepared in an ampoule or vial unit dosage form. The composition may be sterilized or may include auxiliary substance such as preservatives, stabilizers, wetting or emulsifying accelerators, salts and/or buffers for controlling osmotic pressure, and other therapeutically useful substances, and the composition may be formulated according to a common method including a mixing, granulating, or coating method.

Further, the pharmaceutical composition according to the present disclosure may be administered in a pharmaceutically effective amount. As used herein, the term "pharmaceutically effective amount" means an amount sufficient to treat diseases at a reasonable benefit/risk ratio applicable for medical treatment, and the effective dosage level may be determined by various factors including a kind and severity of a patient's disease, activity of a drug, sensitivity to the drug, an administration time, an administration route, and an excretion rate, a treatment period, drugs co-administered, and other factors well known in the medical field. The pharmaceutical composition according to the present disclosure may be administered alone as a single therapeutic agent or in combination together with other therapeutic agents, may be administered sequentially or simultaneously together with known therapeutic agents, and may be administered one or several times. It is important to take into account all of the above factors and to administer a minimum amount that produces a maximum effect while avoiding side effects, which may be easily determined by those skilled in the art.

Specifically, the effective amount of the pharmaceutical composition according to the present disclosure may vary depending on a patient's age, sex, conditions, body weight, an absorption rate, an inactivation rate, and an excretion rate of the active ingredient in the body, a kind of disease, and a drug co-administered, and the pharmaceutical composition may be generally administered at a dose of 0.001 mg to 150 mg, preferably 0.01 mg to 100 mg per 1 kg of body weight daily or every other day once to three times a day. However, the dose may be increased or decreased depending on an administration route, severity of hair loss, sex, body weight, age, etc., and therefore, the dose does not limit the scope of the present disclosure in any way.

Meanwhile, the cosmetic composition of the present disclosure may be prepared in any formulation commonly used in the art, and for example, a solution, a suspension, an emulsion, a paste, a gel, a cream, a lotion, a powder, a soap, a surfactant-containing cleansing, an oil, a powder foundation, an emulsion foundation, a wax foundation, a spray, etc., and more specifically, the formulation may be selected from the group consisting of a hair tonic, a hair conditioner, a hair essence, a hair lotion, a hair-nourishing lotion, a hair shampoo, a hair rinse, a hair treatment, a hair cream, a hair-nourishing cream, a hair moisturizing cream, a hair massage cream, a hair wax, a hair aerosol, a hair pack, a hair-nourishing pack, a hair soap, a hair cleansing foam, a hair oil, a hair drying preparation, a hair preserving and managing preparation, a hair dye, a hair waving preparation, a hair bleaching agent, a hair gel, a hair glaze, a hair dressinger, a hair lacquer, a hair moisturizer, a hair mousse, and a hair spray, but is not limited thereto.

A cosmetically effective carrier included in the cosmetic composition of the present disclosure may be a carrier commonly used in the art depending on the formulation. When the formulation of the present disclosure is a paste, a cream, or a gel, an animal oil, a vegetable oil, a wax, a paraffin, a starch, a tragacanth, a cellulose derivative, polyethylene glycol, silicon, bentonite, silica, talc, or zinc oxide may be used as the carrier component.

When the formulation of the present disclosure is a powder or a spray, lactose, talc, silica, aluminum hydroxide, calcium silicate, or polyamide powder may be used as the carrier component, and particularly, in the case of the spray, a propellant such as chlorofluorohydrocarbon, propane/butane, or dimethyl ether may be additionally included.

When the formulation of the present disclosure is a solution or an emulsion, a solvent, a solubilization agent, or an emulsifying agent may be used as the carrier component, and for example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or sorbitan fatty acid ester may be used.

When the formulation of the present disclosure is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth may be used as the carrier component.

When the formulation of the present disclosure is a surfactant-containing cleansing, aliphatic alcohol sulfate, aliphatic alcohol ether sulfate, sulfosucinnate monoester, isethionate, imidazolium derivatives, methyl taurate, sarcosinate, fatty acid amide ether sulfate, alkyl amido betaine, aliphatic alcohol, fatty acid glyceride, fatty acid diethanolamide, vegetable oil, lanoline derivatives, or ethoxylated glycerol fatty acid ester may be used as the carrier component.

Components included in the cosmetic composition of the present disclosure may include components commonly used in the cosmetic composition, in addition to the active ingredient and carrier component, and for example, the cosmetic composition may include common auxiliary substances such as an antioxidant, stabilizer, a solubilizer, vitamins, a pigment, and a fragrance.

Further, still another aspect of the present disclosure provides a method of preventing hair loss or promoting hair growth, the method including applying or spraying the composition onto the scalp.

Further, still another aspect of the present disclosure provides a method of preventing hair loss or promoting hair growth. Further, still another aspect of the present disclosure provides the method including applying or spraying the composition onto the scalp and a method of producing a large amount of dermal papilla cells and dermal sheath cells, the method including a) isolating dermal papilla cells or dermal sheath cells from the scalp; and b) treating the isolated dermal papilla cells or dermal sheath cells with the anti-IGDCC4 antibody.

The present disclosure has technical characteristics in that proliferation and survival of dermal papilla cells or dermal sheath cells may be promoted by treatment of the anti-IGDCC4 antibody, and the method may further include culturing the produced dermal papilla cells or dermal sheath cells by a method widely known in the art.

Further, still another aspect of the present disclosure provides a cell therapeutic agent for hair regeneration, the cell therapeutic agent including the dermal papilla cells and the dermal sheath cells produced by the above method.

As used herein, the term "cell therapeutic agent" is a cell or a tissue that is prepared from a human by isolation, cultivation, and special manipulation, and used for the purpose of treatment, diagnosis, and prevention, and the cell therapeutic agent refers to a medicine used for treatment, diagnosis, and prevention via a series of actions such as *ex vivo* proliferation or selection of living autologous, homologous, or heterologous cells, or alteration of biological characteristics thereof by other methods in order to restore functions of cells or tissues. With respect to the objects of the present disclosure, the cell therapeutic agent may be administered into dermal papilla cells or dermal sheath cells to regenerate hairs, thereby preventing hair loss and promoting hair growth.

Further, still another aspect of the present disclosure provides use of any one selected from the group consisting of a ligand, an antibody protein, a polypeptide, and a small molecule, each of which specifically binds to the IGDCC4 antigen protein in the preparation of the pharmaceutical or cosmetic composition for preventing hair loss or promoting hair growth.

Further, still another aspect of the present disclosure provides a method of treating hair loss, the method including administering the pharmaceutical composition to a subject, wherein the pharmaceutical composition includes as an active ingredient a pharmaceutically effective amount of any one selected from the group consisting of a ligand, an antibody protein, a polypeptide, and a small molecule, each of which specifically binds to the IGDCC4 antigen protein.

### MODE OF DISCLOSURE

Hereinafter, preferred examples will be provided for better understanding of the present disclosure. However, the following examples are provided only for understanding the present invention more easily, but the content of the present disclosure is not limited thereby.

### Example 1. Identification of dermal papilla cell-binding HARE57 antibody protein

The present inventors for the first time identified a monoclonal antibody protein that specifically binds to IGDCC4 receptor protein to activate dermal papilla cells, and this monoclonal antibody protein was named as HARE57 antibody protein in the following Example.

In this Example, it was intended to examine excellent binding ability between the HARE57 antibody protein of the present disclosure and human dermal papilla cells (DP). In detail, 100,000 human dermal papilla cells under culturing were put in an Eppendorf tube, and washed with staining media (SM: PBS(-)+2% FBS). The HARE57 monoclonal antibody was added thereto to allow primary immunostaining for 30 minutes. After binding with a primary antibody, centrifugation with SM (staining media: PBS(-)+ 2% FBS) was performed and washing was repeated twice in order to remove unbound antibody. Thereafter, anti-mouse-PE secondary antibody was added, and allowed to react at room temperature for 30 minutes. Washing was performed in the same manner as above, and this was used as a sample for flow cytometry. Before flow cytometry, propidium iodide (PI) was added to remove dead cells, and information of 10,000 cells was saved and used as data for flow cytometry. As a control group, a group treated with isotype IgG as a primary antibody was used.

Further, for immunocytostaining, a culture medium was removed from human dermal papilla cells under culturing, and the cells were washed with PBS(-) twice. Then, primary and secondary antibodies were added thereto in the same manner as the above flow cytometry, and binding with cells was examined under a fluorescent microscope. Further, after immunostaining of the antibody, DAPI nuclear staining of the cells was performed to examine binding with cells.

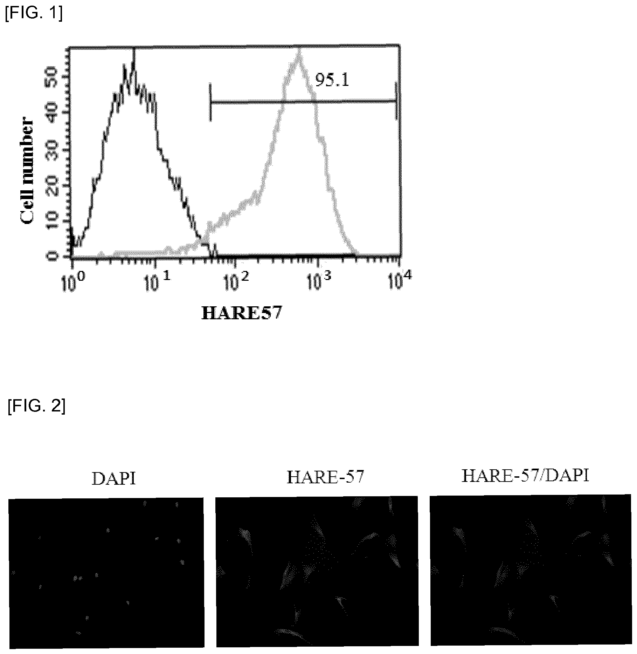

As a result, as shown in FIG. 1, it was confirmed that the HARE57 antibody protein of the present disclosure bound with human dermal papilla cells, and as shown in FIG. 2, it was confirmed by the fluorescent microscope that the HARE57 antibody protein had high ability to bind with human dermal papilla cells.

### Example 2. Examination of specific binding ability of HARE57 antibody protein

In this Example, in order to examine specific binding ability between the HARE57 antibody protein of the present disclosure and dermal papilla cells, a skin tissue of a mouse animal model and human blood cells were used to examine binding characteristics of the HARE57 antibody protein.

### 2-1. Examination of specific binding ability in mouse animal model

Binding characteristics of the HARE57 antibody protein were examined by using a skin tissue section of a mouse. In detail, the skin tissue of C57BL/6 mouse was cryopreserved with OCT solution, and the skin tissue was dissected using a cryotome, and used for immunostaining analysis. To prevent non-specific binding, the tissue sections were allowed to react with a blocking buffer for 30 minutes, and washed with a PBS(-) solution four times. The tissue sections were subjected to primary staining with HARE57 antibody, and washed with PBS(-), followed by immunofluorescent staining with anti-mouse mouse IgG-PE antibody as a secondary antibody. Then, characteristics of binding between the HARE57 antibody protein and the skin tissue were examined under a fluorescent microscope.

Further, in order to analyze characteristics of binding between the HARE57 antibody protein and other tissue cells, the brain, heart, kidney, liver, and lung tissues of the mouse was collected and cryopreserved with OCT solution. Thereafter, the same method as above was performed, and characteristics of binding between the HARE57 antibody protein and various other tissues were examined under a fluorescent microscope.

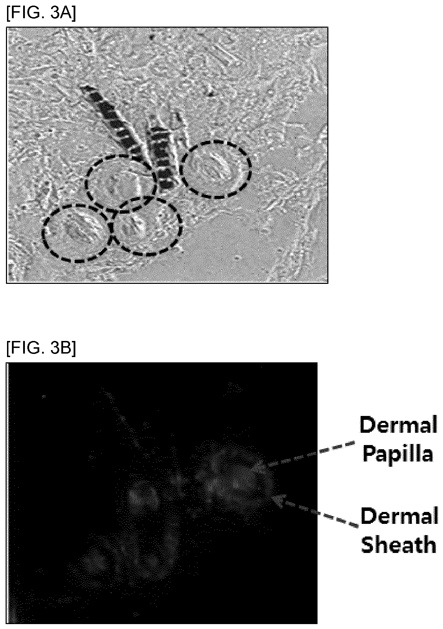

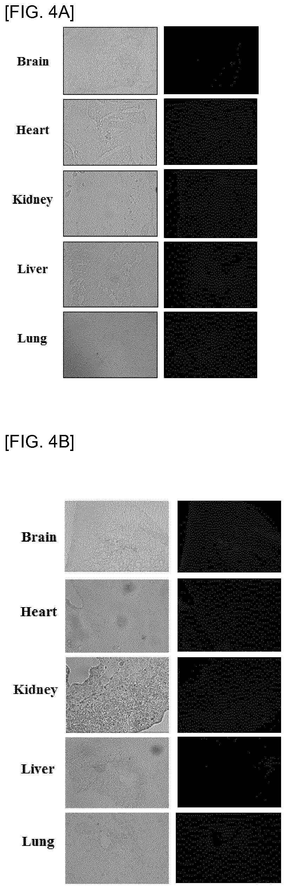

As a result, as shown in FIG. 3, it was confirmed that the HARE57 antibody protein specifically bound with dermal papilla cells in hair follicles, and also specifically bound with dermal sheath cells which have ability to reversibly differentiate to dermal papilla cells, but the HARE57 antibody protein did not bind with other cells of the skin tissue, indicating binding specificity of the HARE57 antibody protein. Further, as shown in FIG. 4, binding of the HARE57 antibody protein was not observed in the brain, heart, kidney, liver, and lung tissues, as in the control group.

### 2-2. Verification of binding ability with human blood cells

Characteristics of binding of the HARE57 antibody protein and blood cells collected from the human blood were examined by flow cytometry. In detail, human blood cells were collected, and a primary cell staining with the HARE57 antibody protein was performed at room temperature for 30 minutes. Centrifugation was performed to remove unbound antibody proteins. The cells were washed with staining media (SM) twice, and then anti-mouse IgG-PE antibody as a secondary antibody was added to perform immunostaining. After reaction with the secondary antibody, centrifugation was performed with SM twice to remove unbound secondary antibody. Thereafter, cell immunization was performed with hCD19-FITC antibody which is a human B immune cell-binding antibody and hCD3-FITC antibody which is a T immune cell-binding antibody, and then used as a sample for flow cytometry. Upon flow cytometry, staining with propidium iodide (PI) was performed in order to remove dead blood cells, and information of 10,000 cells per sample was saved and used as data for flow cytometry.

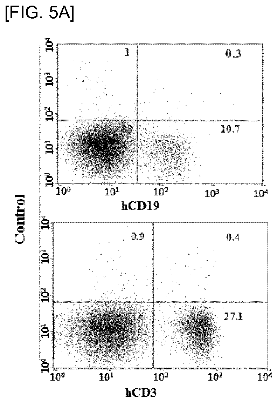

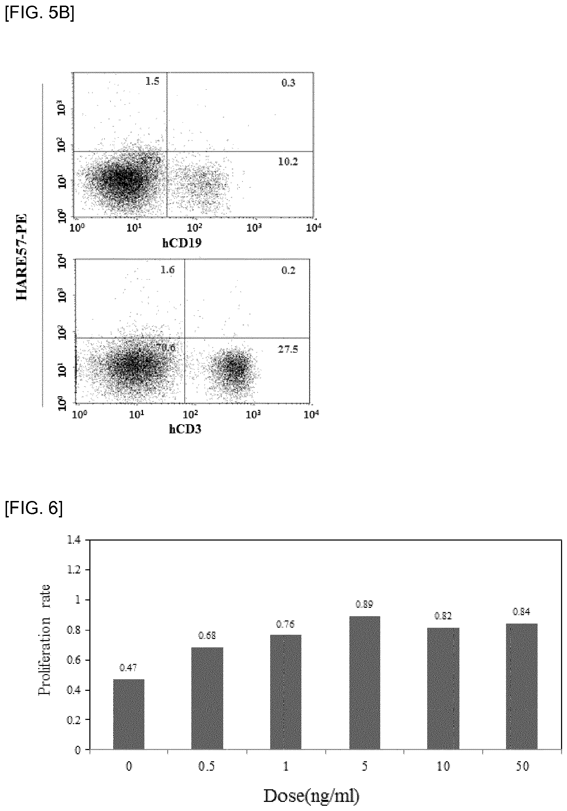

As a result, as shown in FIG. 5, binding of the HARE57 antibody protein with the human blood cells was not observed, as in the control group. The results of Examples 1 and 2-1 taken together showed that the HARE57 antibody protein of the present disclosure has ability to specifically bind with dermal papilla cells and dermal sheath cells.

### Example 3. Examination of dermal papilla cell proliferation-promoting effect of HARE57 antibody protein

In this Example, in order to examine changes in proliferation of dermal papilla cells according to treatment with the HARE57 antibody protein of the present disclosure, cell regulatory physiological functions of the HARE57 antibody protein in human dermal papilla cells (DP) were analyzed. In detail, human dermal papilla cells under culturing were collected and washed with DMEM twice. Thereafter, 10,000 cells were plated in each well of a 96 well plate and different concentrations of HARE57 antibody protein (0, 5, 1, 5, 10, 50 ng/ml) were added to the DMEM medium, followed by culturing at 37°C for 72 hours. After culture, changes in the proliferation rate of the human dermal papilla cells according to treatment with the HARE57 antibody protein were examined by using an MTT assay kit.

Further, in order to investigate activity of human dermal papilla cells, the human dermal papilla cells under culturing were cultured in FBS free DMEM medium supplemented with the HARE57 antibody protein (100 ng/ml), and then morphological changes of human dermal papilla cells were examined. As a control group, a group which was not treated with the HARE57 antibody protein was used.

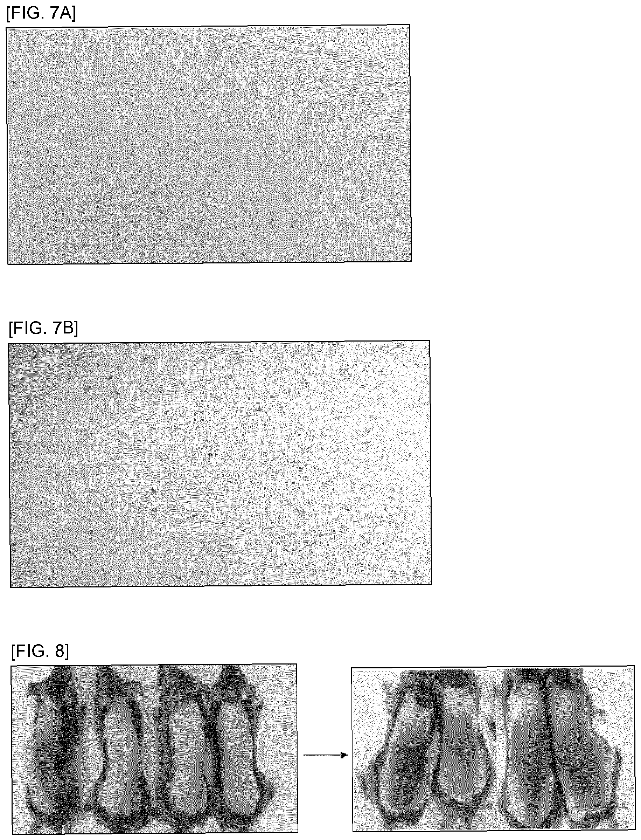

As a result, as shown in FIGS. 6 and 7, the proliferation rate was significantly increased and various morphological changes were observed in the HARE57 antibody protein-treated group, as compared with the control group, indicating that the HARE57 antibody protein of the present disclosure stimulates activation of human dermal papilla cells to improve proliferation ability thereof.

### Example 4. Examination of hair growth-promoting effect in mouse animal model

In this Example, it was intended to examine *in vivo* hair growth-promoting effects according to treatment of mouse animal models with the HARE57 antibody protein of the present disclosure, based on the results of Example 3. In particular, in order to investigate hair growth-promoting effects according to various causes of hair loss, normal mice, gene-deficient nude mice, and stress-induced hair loss mice were subjected to experiments.

In detail, in order to investigate *in vivo* hair growth-promoting effects according to treatment of normal mice with the HARE57 antibody protein, 6-week-old C3H mice were shaved and a formulation containing the HARE57 antibody protein (100 ng/ml) was applied once a day for 2 weeks, and hair growth-promoting effects were examined with the naked eye. Further, nude mice (BALB/ c-nude) with atrichia caused by congenital genetic deficiency were used to investigate therapeutic effects on hair loss caused by gene deficiency. The nude mice are known to have a small number of dermal papilla cells in hair follicles and zigzag type hairs in the subcutaneous tissue. After applying the formulation containing the HARE57 antibody protein (100 ng/ml) once a day for 17 days, hair growth-promoting effects were examined with the naked eye. As a control group, a group which was not treated with the HARE57 antibody protein was used.

Furthermore, in order to investigate therapeutic effects on stress-induced hair loss, hair loss-induced mice were selected from mice raised under overcrowded environment of a limited cage. The selected mice were subjected to the above experiments and the effects were examined after 14 days.

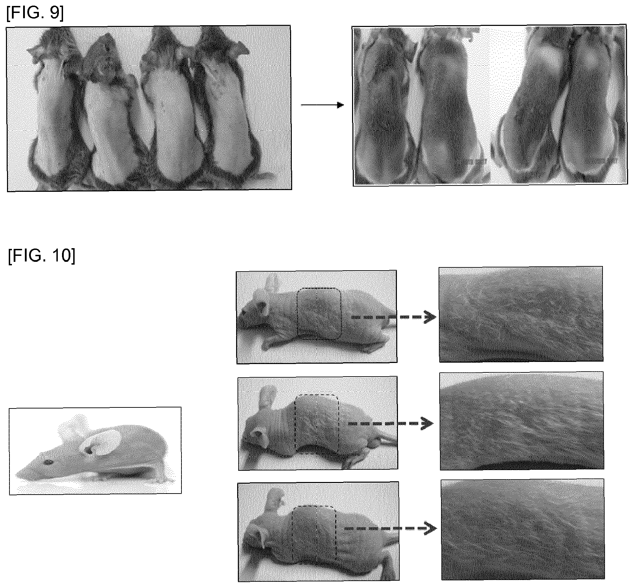

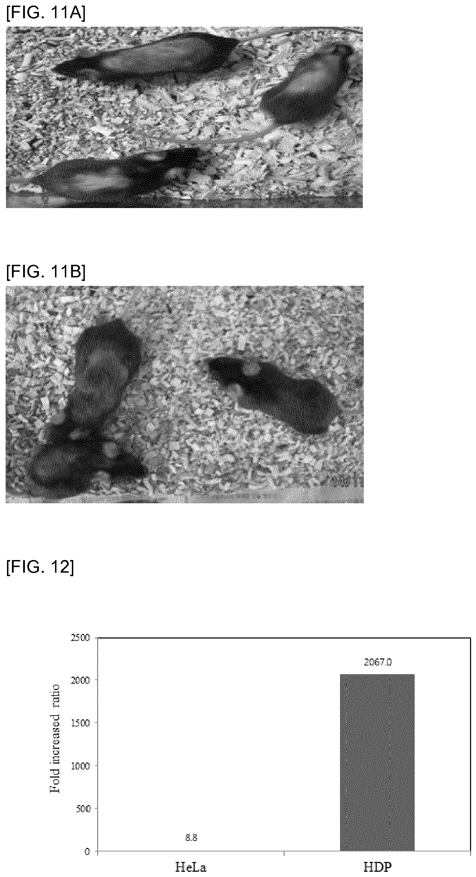

As a result, as shown in FIGS. 9 and 10, remarkably excellent hair growth-promoting effects were observed in the HARE57 antibody protein-treated groups, as compared with the control groups which were not treated with the HARE57 antibody protein. As shown in FIG. 11, in the gene-deficient nude mice, hairs developed to a guard type from a zigzag type which is a hair type in the general nude mice, and grew out of the skin, indicating that hair regeneration was promoted. As shown in FIG. 12, the hair growth-promoting effect of the HARE57 antibody protein was also observed in the stress-induced hair loss mice. These results suggest that the HARE57 antibody protein of the present disclosure activates dermal papilla cells to promote hair growth, and therefore, it may be usefully applied to the treatment of hair loss.

### Example 5. Examination of hair growth-promoting mechanism of HARE57 antibody protein

In this Example, in order to examine a mechanism of the hair growth-promoting effect as confirmed in Examples 3 and 4, proteins specifically binding with the HARE57 antibody protein of the present disclosure were identified, and changes in signaling mechanisms related therewith and related factors were analyzed.

### 5-1. Examination of target receptor proteins and signaling mechanisms

Experiments were conducted, based on the assumption that the HARE57 antibody protein induces activation of dermal papilla cells through signaling by specific binding with cell membrane proteins of dermal papilla cells. Dermal papilla cells expressing HARE57 antibody-binding cell membrane protein and HeLa cells expressing no HARE57 antibody-binding cell membrane protein were used to perform microarray, and mRNA levels were compared therebetween. Further, in order to investigate specific binding of the HARE57 antibody protein with IGDCC4 proteins identified by the experiment, binding of extracellular regions of the IGDCC4 proteins with the HARE57 antibody protein was analyzed by ELISA.

Furthermore, in order to investigate a cell signaling mechanism through the IGDCC4 receptor protein binding with the cell membrane protein of dermal papilla cells, human dermal papilla cells cultured *in vitro* were treated with HARE57 antibody protein (100 ng/ml), and cultured for 48 hours. Then, protein expression patterns in 1000 cells were analyzed by a protein array. As a control group, a group which was not treated with the HARE57 antibody protein was used.

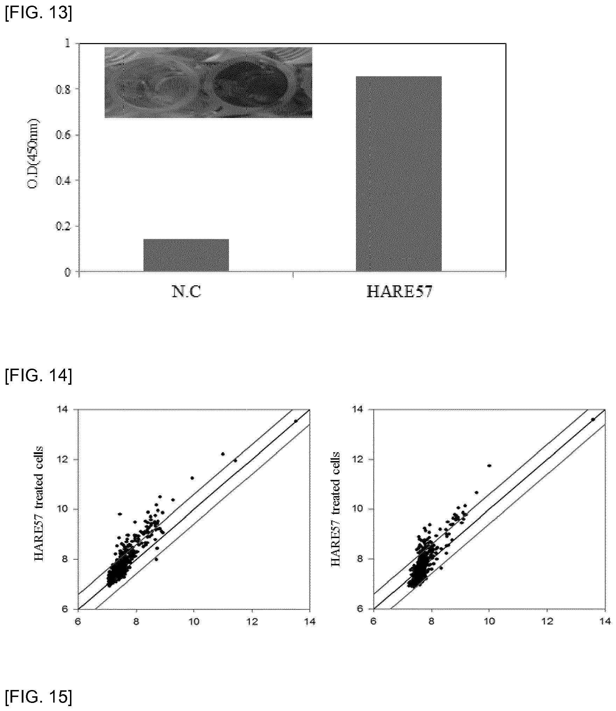

As a result, as shown in FIGS. 12 and 13, from various cell membrane proteins of dermal papilla cells, IGDCC4 (NOPE, DDM36, KIAA1628) which were about 235 times highly expressed were identified in dermal papilla cells, whereas none of them were expressed in HeLa cells. Specific binding of the IGDCC4 protein and the HARE57 antibody protein was also confirmed by ELISA. As shown in FIG. 14, it was confirmed that expression of the proteins involved in cell proliferation and various cell regulatory factors was increased by stimulation of the HARE57 antibody protein, suggesting that the HARE57 antibody protein of the present disclosure specifically binds with IGDCC4 protein which is one of cell membrane proteins of dermal papilla cells to increase expression of proteins related with proliferation of dermal papilla cells, thereby promoting hair growth.

### 5-2. Comparison of expression levels of hair growth-related regulatory factors

Based on the protein array results of Example 5-1, expression of the proteins or regulatory factors involved in hair growth or hair regeneration was compared. In detail, expression of ALPP which is a representative stem cell biomarker, the FGF, Wnt, TGF, PDGF, ECM, TNF family proteins which are involved in hair regeneration, cell proliferation-related proteins, and blood cell regulatory factors was compared. As a control group, a group which was not treated with the HARE57 antibody protein was used.

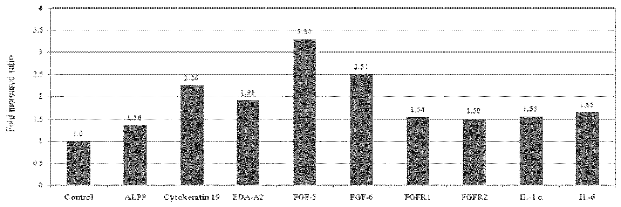

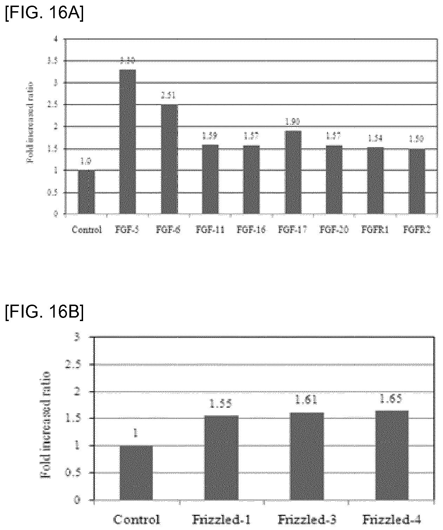

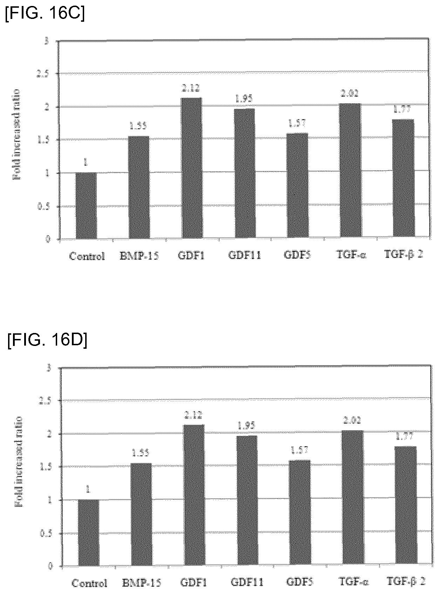

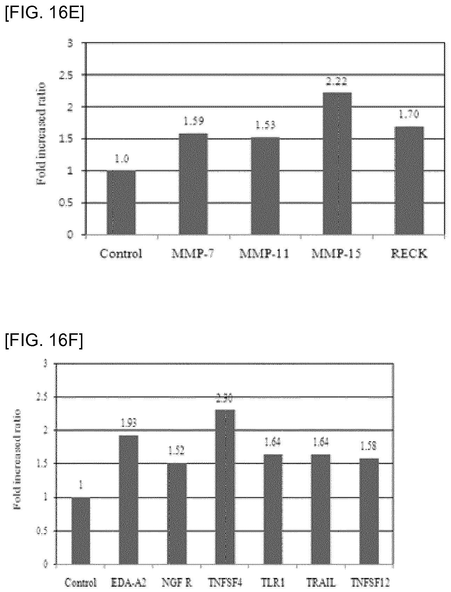

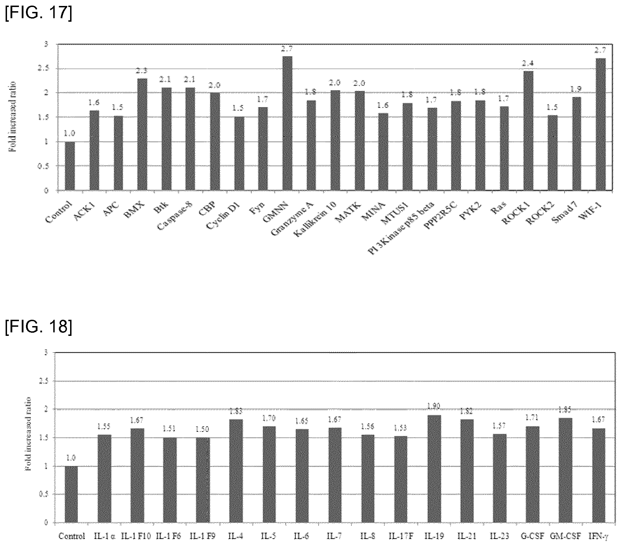

As a result, as shown in FIG. 15, ALPP, cytokeratin19, EDA-A2, FGF-5, FGF-6, FGFR1, FGFR2, IL-1α, and IL-6 were found to be about 1.5 times highly expressed, as compared with the control groups, and in particular, increased expression of ALPP which is a representative stem cell biomarker suggests that the HARE57 antibody protein influences stem cell function of dermal papilla cells. Further, as shown in FIG. 16, increased expression of the FGF family proteins (FGF-5, FGF-6, FGF-11, FGF-16, FGF-17, FGF-20, FGFR1 and FGFR2) and the Wnt family protein receptors (Frizzled-1, Frizzled-3, and Frizzled-4) was confirmed and increased expression of the TGF family proteins (BMP-15, GDF1, GDF11, GDF5, TGF-α, and TGF-β2), the PDGF family proteins (HGFR and VEGF R2), the ECM family proteins (MMP-7, MMP-11, MMP-15, and RECK), and the TNF family proteins (EDA-A2, NGFR, TNFSF4, TLR1, TRAIL, and TNFSF12) was also confirmed. Furthermore, as shown in FIGS. 17 and 18, increased expression of GMNN, ROCK1, WIF-1 which are cell proliferation-related proteins and increased expression of IL-1, IL-4, IL-6, IL-7, IL-8, IL-21, IL-23, G-CSF, GM-CSF, and IFN-α which are blood cell regulatory factors were confirmed. Accordingly, it was confirmed that treatment of the HARE57 antibody protein of the present disclosure promotes proliferation of dermal papilla cells and hair growth by increasing expression of various regulatory factors.

Although the above description of the present disclosure has been described for illustrative purposes, it will be understood by those skilled in the art to which the present disclosure pertains that the present disclosure may be implemented in a different specific form without changing the technical spirit or essential characteristics thereof. Therefore, it should be understood that the above embodiments are not limitative, but illustrative in all aspects.

## Claims

1. An IGDCC4 antigen protein consisting of an amino acid sequence represented by SEQ ID NO: 17.

2. An anti-IGDCC4 antibody or a fragment thereof comprising an antigen-binding site which specifically binds to the IGDCC4 antigen protein of claim 1.

3. The anti-IGDCC4 antibody or fragment thereof comprising an antigen-binding site of claim 2, wherein the antibody comprises a heavy chain variable region comprising one or more selected from the group consisting of HCDR1 consisting of an amino acid sequence represented by SEQ ID NO: 1, HCDR2 consisting of an amino acid sequence represented by SEQ ID NO: 2, and HCDR3 consisting of an amino acid sequence represented by SEQ ID NO: 3; and a light chain variable region comprising one or more selected from the group consisting of LCDR1 consisting of an amino acid sequence represented by SEQ ID NO: 4, LCDR2 consisting of an amino acid sequence represented by SEQ ID NO: 5, and LCDR3 consisting of an amino acid sequence represented by SEQ ID NO: 6.

4. The anti-IGDCC4 antibody or fragment thereof comprising an antigen-binding site of claim 2, wherein the antibody comprises a heavy chain variable region consisting of an amino acid sequence represented by SEQ ID NO: 7 and a light chain variable region consisting of an amino acid sequence represented by SEQ ID NO: 8.

5. The anti-IGDCC4 antibody or fragment thereof comprising an antigen-binding site of claim 2, wherein the antibody comprises a heavy chain variable region comprising one or more selected from the group consisting of HCDR1 consisting of a polynucleotide sequence represented by SEQ ID NO: 9, HCDR2 consisting of a polynucleotide sequence represented by SEQ ID NO: 10, and HCDR3 consisting of a polynucleotide sequence represented by SEQ ID NO: 11; and a light chain variable region comprising one or more selected from the group consisting of LCDR1 consisting of a polynucleotide sequence represented by SEQ ID NO: 12, LCDR2 consisting of a polynucleotide sequence represented by SEQ ID NO: 13, and LCDR3 consisting of a polynucleotide sequence represented by SEQ ID NO: 14.

6. The anti-IGDCC4 antibody or fragment thereof comprising an antigen-binding site of claim 2, wherein the antibody comprises a heavy chain variable region consisting of a polynucleotide sequence represented by SEQ ID NO: 15 and a light chain variable region consisting of a polynucleotide sequence represented by SEQ ID NO: 16.

7. The anti-IGDCC4 antibody or fragment thereof comprising an antigen-binding site of claim 2, wherein the antibody is a monoclonal antibody or a polyclonal antibody.

8. The anti-IGDCC4 antibody or fragment thereof comprising an antigen-binding site of any one of claims 1 to 7, wherein the antibody comprises any one light chain or heavy chain variable region selected from the group consisting of Fab, Fab', F(ab')₂, dsFv, scFv, sc(Fv)₂, IgNAR, hcIgG, (scFv-SA)₄, [sc(Fv)₂]₂, minibody, diabody, tribody, nanobody, and camelbody.

9. A method of preparing an anti-IGDCC4 antibody, the method comprising injecting the IGDCC4 antigen protein of claim 1 into an animal.

10. An expression vector comprising an anti-IGDCC4 antibody or antigen-binding site thereof comprising isolated polynucleotides encoding amino acid sequences consisting of SEQ ID NO: 7 and SEQ ID NO: 8.

11. A host cell which is transformed *in vitro* with the expression vector for producing the anti-IGDCC4 antibody or fragment thereof comprising an antigen-binding site of claim 10

12. The host cell of claim 11, wherein the host cell is any one selected from the group consisting of bacteria (E. *coli*)*,* yeast, CHO cells, F2N cells, HEK293 cells, and antibody-producing hybridoma cells.

13. A method of producing an anti-IGDCC4 antibody *in vitro,* the method comprising infecting host cells with the expression vector of claim 10.

14. A pharmaceutical composition for preventing hair loss or promoting hair growth, the composition comprising any one selected from the group consisting of a ligand, an antibody protein, a polypeptide, and a small molecule, each of which specifically binds to the IGDCC4 antigen protein of claim 1.

15. The pharmaceutical composition of claim 14, wherein the antibody protein comprises a heavy chain variable region comprising one or more selected from the group consisting of HCDR1 consisting of an amino acid sequence represented by SEQ ID NO: 1, HCDR2 consisting of an amino acid sequence represented by SEQ ID NO: 2, and HCDR3 consisting of an amino acid sequence represented by SEQ ID NO: 3; and a light chain variable region comprising one or more selected from the group consisting of LCDR1 consisting of an amino acid sequence represented by SEQ ID NO: 4, LCDR2 consisting of an amino acid sequence represented by SEQ ID NO: 5, and LCDR3 consisting of an amino acid sequence represented by SEQ ID NO: 6.

16. The pharmaceutical composition of claim 14, wherein the composition specifically binds to dermal papilla cells or dermal sheath cells to thereby promote proliferation of the dermal papilla cells.

17. The pharmaceutical composition of claim 14, wherein the composition is for preparation into a formulation selected from the group consisting of a cream, a gel, a patch, a spray, an ointment, a plaster, a lotion, a liniment, a paste, and a cataplasma.

18. A cosmetic composition for preventing hair loss or promoting hair growth, the composition comprising any one selected from the group consisting of a ligand, an antibody protein, a polypeptide, and a small molecule, each of which specifically binds to the IGDCC4 antigen protein of claim 1.

19. The cosmetic composition of claim 18, wherein the antibody protein comprises a heavy chain variable region comprising one or more selected from the group consisting of HCDR1 consisting of an amino acid sequence represented by SEQ ID NO: 1, HCDR2 consisting of an amino acid sequence represented by SEQ ID NO: 2, and HCDR3 consisting of an amino acid sequence represented by SEQ ID NO: 3; and a light chain variable region comprising one or more selected from the group consisting of LCDR1 consisting of an amino acid sequence represented by SEQ ID NO: 4, LCDR2 consisting of an amino acid sequence represented by SEQ ID NO: 5, and LCDR3 consisting of an amino acid sequence represented by SEQ ID NO: 6.

20. The cosmetic composition of claim 18, wherein the composition is for preparation into a formulation selected from the group consisting of a hair tonic, a hair conditioner, a hair essence, a hair lotion, a hair-nourishing lotion, a hair shampoo, a hair rinse, a hair treatment, a hair cream, a hair-nourishing cream, a hair moisturizing cream, a hair massage cream, a hair wax, a hair aerosol, a hair pack, a hair-nourishing pack, a hair soap, a hair cleansing foam, a hair oil, a hair drying preparation, a hair preserving and managing preparation, a hair dye, a hair waving preparation, a hair bleaching agent, a hair gel, a hair glaze, a hair dressinger, a hair lacquer, a hair moisturizer, a hair mousse, and a hair spray.

21. A method of preventing hair loss or promoting hair growth, the method comprising applying or spraying the composition of claim 18 onto the scalp.

22. A method of mass-producing dermal papilla cells and dermal sheath cells, the method comprising:
a) isolating dermal papilla cells or dermal sheath cells from the scalp; and
b) treating the isolated dermal papilla cells or dermal sheath cells with the anti-IGDCC4 antibody of any one of claims 2 to 8.

23. A cell therapeutic agent for hair regeneration, the cell therapeutic agent comprising dermal papilla cells and dermal sheath cells produced by the method of claim 22.

24. Use of the anti-IGDCC4 antibody or fragment thereof comprising the antigen-binding site of claim 10 in preventing hair loss or promoting hair growth.

25. Use of any one selected from the group consisting of a ligand, an antibody protein, a polypeptide, and a small molecule, each of which specifically binds to the IGDCC4 antigen protein of claim 1, in the preparation of a pharmaceutical or cosmetic composition for preventing hair loss or promoting hair growth.

26. A method of treating hair loss, the method comprising administering a pharmaceutical composition to a subject, wherein the pharmaceutical composition comprises, as an active ingredient, a pharmaceutically effective amount of any one selected from the group consisting of a ligand, an antibody protein, a polypeptide, and a small molecule, each of which specifically binds to the IGDCC4 antigen protein of claim 1.


<110>     KIM, dong ku

<120>     Antibody for specific binding and functional activator to hair
          Dermal Papilla cell and use thereof

<130>     MPCT16-049

<150>     KR 10/2015/0158897
<151>     2015-11-12

<160>     17

<170>     KoPatentIn 3.0

<210>     1
<211>     8
<212>     PRT
<213>     Artificial Sequence

<220>
<223>     anti-IGDCC4 antibody(HARE57) heavy chain CDR1 region

<400>     1
Gly Phe Thr Phe Ser Asn Tyr Trp
  1               5

<210>     2
<211>     10
<212>     PRT
<213>     Artificial Sequence

<220>
<223>     anti-IGDCC4 antibody(HARE57) heavy chain CDR2 region

<400>     2
Ile Arg Leu Lys Ser Asp Asn Tyr Ala Thr
  1               5                   10

<210>     3
<211>     7
<212>     PRT
<213>     Artificial Sequence

<220>
<223>     anti-IGDCC4 antibody(HARE57) heavy chain CDR3 region

<400>     3
Thr Ala Gly Phe Ala Tyr Trp
  1               5

<210>     4
<211>     10
<212>     PRT
<213>     Artificial Sequence

<220>
<223>     anti-IGDCC4 antibody(HARE57) light chain CDR1 region

<400>    4
Lys Ser Val Ser Thr Ser Gly Tyr Ser Tyr
1               5                   10

<210>    5
<211>    7
<212>    PRT
<213>    Artificial Sequence

<220>
<223>    anti-IGDCC4 antibody(HARE57) light chain CDR2 region

<400>    5
Ile Tyr Leu Val Ser Asn Leu
1               5

<210>    6
<211>    6
<212>    PRT
<213>    Artificial Sequence

<220>
<223>    anti-IGDCC4 antibody(HARE57) light chain CDR3 region

<400>    6
Gln His Ile Arg Glu Leu
1               5

<210>    7
<211>    157
<212>    PRT
<213>    Artificial Sequence

<220>
<223>    anti-IGDCC4 antibody(HARE57) heavy chain peptide sequence

<400>    7
Met Asn Phe Gly Leu Ser Trp Val Phe Ile Ile Val Leu Leu Lys Gly
1               5                   10                  15

Val Gln Ser Glu Val Lys Leu Glu Glu Ser Gly Gly Gly Leu Val Gln
            20                  25                  30

Pro Gly Gly Ser Met Lys Leu Ser Cys Val Ala Ser Gly Phe Thr Phe
        35                  40                  45

Ser Asn Tyr Trp Met Asn Trp Val Arg Gln Ser Pro Glu Lys Gly Leu
    50                  55                  60

Glu Trp Val Ala Gln Ile Arg Leu Lys Ser Asp Asn Tyr Ala Thr His
65                  70                  75                  80

Tyr Ala Glu Ser Val Lys Gly Arg Phe Thr Ile Ser Arg Asp Asp Ser
                85                  90                  95

Lys Ser Ser Val Tyr Leu Gln Met Asn Asn Leu Arg Ala Glu Asp Thr
            100                 105                 110

Gly Ile Tyr Tyr Cys Thr Ala Gly Phe Ala Tyr Trp Gly Gln Gly Thr
        115                 120                 125

Leu Val Thr Val Ser Ala Ala Ser Pro Thr Leu Val Thr Val Ser Ala
    130                 135                 140

Ala Ser Pro Thr Thr Val Thr Val Ser Ser Ala Ser Pro
145                 150                 155


<210>    8
<211>    128
<212>    PRT
<213>    Artificial Sequence

<220>
<223>    anti-IGDCC4 antibody(HARE57) light chain peptide sequence


<400>    8
Met Glu Thr Asp Thr Leu Leu Leu Trp Val Leu Leu Leu Trp Val Pro
  1               5                  10                  15

Gly Ser Thr Gly Asp Ile Val Leu Thr Gln Ser Pro Ala Ser Leu Ala
            20                  25                  30

Val Ser Leu Gly Gln Arg Ala Thr Ile Ser Tyr Arg Ala Ser Lys Ser
        35                  40                  45

Val Ser Thr Ser Gly Tyr Ser Tyr Met His Trp Asn Gln Gln Lys Pro
     50                  55                  60

Gly Gln Pro Pro Arg Leu Leu Ile Tyr Leu Val Ser Asn Leu Glu Ser
 65                  70                  75                  80

Gly Val Pro Ala Arg Phe Ser Gly Ser Gly Ser Gly Thr Asp Phe Thr
                85                  90                  95

Leu Asn Ile His Pro Val Glu Glu Glu Asp Ala Ala Thr Tyr Tyr Cys
            100                 105                 110

Gln His Ile Arg Glu Leu Thr Arg Ser Glu Gly Gly Pro Ser Trp Ser
        115                 120                 125




<210>    9
<211>    24
<212>    DNA
<213>    Artificial Sequence

<220>
<223>    anti-IGDCC4 antibody(HARE57) heavy chain CDR1 region nucleotide


<400>    9
ggattcactt tcagtaacta ctgg                                              24

<210>    10
<211>    30
<212>    DNA
<213>    Artificial Sequence

<220>
<223>    anti-IGDCC4 antibody(HARE57) heavy chain CDR2 region nucleotide


<400>    10
attagattga aatctgataa ttatgcaaca                                        30


<210>    11
<211>    21
<212>    DNA
<213>    Artificial Sequence

<220>
<223>    anti-IGDCC4 antibody(HARE57) heavy chain CDR3 region nucleotide


<400>    11
acagctgggt ttgcttactg g                                                 21


<210>    12
<211>    30
<212>    DNA
<213>    Artificial Sequence

<220>
<223>    anti-IGDCC4 antibody(HARE57) light chain CDR1 region nucleotide


<400>    12
aaaagtgtca gtacatctgg ctatagttat                                        30


<210>    13
<211>    21
<212>    DNA
<213>    Artificial Sequence

<220>
<223>    anti-IGDCC4 antibody(HARE57) light chain CDR2 region nucleotide


<400>    13
atctatcttg tatccaacct a                                                 21


<210>    14
<211>    18
<212>    DNA
<213>    Artificial Sequence

<220>
<223>    anti-IGDCC4 antibody(HARE57) light chain CDR3 region nucleotide


<400>    14
cagcacatta gggagctt                                                     18

<210>    15
<211>    471
<212>    DNA
<213>    Artificial Sequence

<220>
<223>    anti-IGDCC4 antibody(HARE57) heavy chain nucleotide sequence


<400>    15
atgaacttcg ggttgagctg ggtttttatt attgttcttt taaaaggggt ccagagtgaa      60

gtgaagcttg aggagtctgg aggaggcttg gtgcaacctg gaggatccat gaaactctcc     120

tgtgttgcct ctggattcac tttcagtaac tactggatga actgggtccg ccagtctcca     180

gagaaggggc ttgagtgggt tgctcaaatt agattgaaat ctgataatta tgcaacacat     240

tatgcggagt ctgtgaaagg gaggttcacc atctcaagag atgattccaa aagtagtgtc     300

tacctgcaaa tgaacaactt aagggctgaa gacactggaa tttattactg cacagctggg     360

tttgcttact ggggccaagg gactctggtc actgtctctg cagctagccc cactctggtc     420

actgtctctg cagctagccc caccacggtc accgtctcct cagctagccc c              471


<210>    16
<211>    384
<212>    DNA
<213>    Artificial Sequence

<220>
<223>    anti-IGDCC4 antibody(HARE57) light chain nucleotide sequence


<400>    16
atggagacag acacactcct gctatgggta ctgctgctct gggttccagg ttccactggt      60

gacattgtgc tgacacagtc tcctgcttcc ttagctgtat ctctggggca gagggccacc     120

atctcataca gggccagcaa aagtgtcagt acatctggct atagttatat gcactggaac     180

caacagaaac caggacagcc acccagactc ctcatctatc ttgtatccaa cctagaatct     240

ggggtccctg ccaggttcag tggcagtggg tctgggacag acttcaccct caacatccat     300

cctgtggagg aggaggatgc tgcaacctat tactgtcagc acattaggga gcttacacgt     360

tcggaggggg gaccaagctg gagc                                            384


<210>    17
<211>    1250
<212>    PRT
<213>    Artificial Sequence

<220>
<223>    IGDCC4 antigen protein

<400>    17
Met Ala Arg Gly Asp Ala Gly Arg Gly Arg Gly Leu Leu Ala Leu Thr
1               5                   10                  15

Phe Cys Leu Leu Ala Ala Arg Gly Glu Leu Leu Leu Pro Gln Glu Thr
            20                  25                  30

Thr Val Glu Leu Ser Cys Gly Val Gly Pro Leu Gln Val Ile Leu Gly
        35                  40                  45

Pro Glu Gln Ala Ala Val Leu Asn Cys Ser Leu Gly Ala Ala Ala Ala
    50                  55                  60

Gly Pro Pro Thr Arg Val Thr Trp Ser Lys Asp Gly Asp Thr Leu Leu
65                  70                  75                  80

Glu His Asp His Leu His Leu Leu Pro Asn Gly Ser Leu Trp Leu Ser
                85                  90                  95

Gln Pro Leu Ala Pro Asn Gly Ser Asp Glu Ser Val Pro Glu Ala Val
            100                 105                 110

Gly Val Ile Glu Gly Asn Tyr Ser Cys Leu Ala His Gly Pro Leu Gly
        115                 120                 125

Val Leu Ala Ser Gln Thr Ala Val Val Lys Leu Ala Thr Leu Ala Asp
    130                 135                 140

Phe Ser Leu His Pro Glu Ser Gln Thr Val Glu Glu Asn Gly Thr Ala
145                 150                 155                 160

Arg Phe Glu Cys His Ile Glu Gly Leu Pro Ala Pro Ile Ile Thr Trp
                165                 170                 175

Glu Lys Asp Gln Val Thr Leu Pro Glu Glu Pro Arg Leu Ile Val Leu
            180                 185                 190

Pro Asn Gly Val Leu Gln Ile Leu Asp Val Gln Glu Ser Asp Ala Gly
        195                 200                 205

Pro Tyr Arg Cys Val Ala Thr Asn Ser Ala Arg Gln His Phe Ser Gln
    210                 215                 220

Glu Ala Leu Leu Ser Val Ala His Arg Gly Ser Leu Ala Ser Thr Arg
225                 230                 235                 240

Gly Gln Asp Val Val Ile Val Ala Ala Pro Glu Asn Thr Thr Val Val
                245                 250                 255

Ser Gly Gln Ser Val Val Met Glu Cys Val Ala Ser Ala Asp Pro Thr
            260                 265                 270

Pro Phe Val Ser Trp Val Arg Gln Asp Gly Lys Pro Ile Ser Thr Asp
        275                 280                 285

Val Ile Val Leu Gly Arg Thr Asn Leu Leu Ile Ala Asn Ala Gln Pro
    290                 295                 300

Trp His Ser Gly Val Tyr Val Cys Arg Ala Asn Lys Pro Arg Thr Arg
305                 310                 315                 320

Asp Phe Ala Thr Ala Ala Ala Glu Leu Arg Val Leu Ala Ala Pro Ala
                325                 330                 335

Ile Thr Gln Ala Pro Glu Ala Leu Ser Arg Thr Arg Ala Ser Thr Ala
            340                 345                 350

Arg Phe Val Cys Arg Ala Ser Gly Glu Pro Arg Pro Ala Leu Arg Trp
        355                 360                 365

Leu His Asn Gly Ala Pro Leu Arg Pro Asn Gly Arg Val Lys Val Gln
    370                 375                 380

Gly Gly Gly Gly Ser Leu Val Ile Thr Gln Ile Gly Leu Gln Asp Ala
385                 390                 395                 400

Gly Tyr Tyr Gln Cys Val Ala Glu Asn Ser Ala Gly Met Ala Cys Ala
                405                 410                 415

Ala Ala Ser Leu Ala Val Val Val Arg Glu Gly Leu Pro Ser Ala Pro
            420                 425                 430

Thr Arg Val Thr Ala Thr Pro Leu Ser Ser Ser Ala Val Leu Val Ala
        435                 440                 445

Trp Glu Arg Pro Glu Met His Ser Glu Gln Ile Ile Gly Phe Ser Leu
    450                 455                 460

His Tyr Gln Lys Ala Arg Gly Met Asp Asn Val Glu Tyr Gln Phe Ala
465                 470                 475                 480

Val Asn Asn Asp Thr Thr Glu Leu Gln Val Arg Asp Leu Glu Pro Asn
                485                 490                 495

Thr Asp Tyr Glu Phe Tyr Val Val Ala Tyr Ser Gln Leu Gly Ala Ser
            500                 505                 510

Arg Thr Ser Thr Pro Ala Leu Val His Thr Leu Asp Asp Val Pro Ser
        515                 520                 525

Ala Ala Pro Gln Leu Ser Leu Ser Ser Pro Asn Pro Ser Asp Ile Arg
    530                 535                 540

Val Ala Trp Leu Pro Leu Pro Pro Ser Leu Ser Asn Gly Gln Val Val
545                 550                 555                 560

Lys Tyr Lys Ile Glu Tyr Gly Leu Gly Lys Glu Asp Gln Ile Phe Ser
                565                 570                 575

Thr Glu Val Arg Gly Asn Glu Thr Gln Leu Met Leu Asn Ser Leu Gln
            580                 585                 590

Pro Asn Lys Val Tyr Arg Val Arg Ile Ser Ala Gly Thr Ala Ala Gly
        595                 600                 605

Phe Gly Ala Pro Ser Gln Trp Met His His Arg Thr Pro Ser Met His
    610                 615                 620

Asn Gln Ser His Val Pro Phe Ala Pro Ala Glu Leu Lys Val Gln Ala
625                 630                 635                 640

Lys Met Glu Ser Leu Val Val Ser Trp Gln Pro Pro Pro His Pro Thr
                645                 650                 655

Gln Ile Ser Gly Tyr Lys Leu Tyr Trp Arg Glu Val Gly Ala Glu Glu
            660                 665                 670

Glu Ala Asn Gly Asp Arg Leu Pro Gly Gly Arg Gly Asp Gln Ala Trp
        675                 680                 685

Asp Val Gly Pro Val Arg Leu Lys Lys Lys Val Lys Gln Tyr Glu Leu
    690                 695                 700

Thr Gln Leu Val Pro Gly Arg Leu Tyr Glu Val Lys Leu Val Ala Phe
705                 710                 715                 720

Asn Lys His Glu Asp Gly Tyr Ala Ala Val Trp Lys Gly Lys Thr Glu
                725                 730                 735

Lys Ala Pro Ala Pro Asp Met Pro Ile Gln Arg Gly Pro Pro Leu Pro
            740                 745                 750

Pro Ala His Val His Ala Glu Ser Asn Ser Ser Thr Ser Ile Trp Leu
        755                 760                 765

Arg Trp Lys Lys Pro Asp Phe Thr Thr Val Lys Ile Val Asn Tyr Thr
    770                 775                 780

Val Arg Phe Ser Pro Trp Gly Leu Arg Asn Ala Ser Leu Val Thr Tyr
785                 790                 795                 800

Tyr Thr Ser Ser Gly Glu Asp Ile Leu Ile Gly Gly Leu Lys Pro Phe
                805                 810                 815

Thr Lys Tyr Glu Phe Ala Val Gln Ser His Gly Val Asp Met Asp Gly
            820                 825                 830

Pro Phe Gly Ser Val Val Glu Arg Ser Thr Leu Pro Asp Arg Pro Ser
        835                 840                 845

Thr Pro Pro Ser Asp Leu Arg Leu Ser Pro Leu Thr Pro Ser Thr Val
    850                 855                 860

Arg Leu His Trp Cys Pro Pro Thr Glu Pro Asn Gly Glu Ile Val Glu
865                 870                 875                 880

Tyr Leu Ile Leu Tyr Ser Ser Asn His Thr Gln Pro Glu His Gln Trp
                885                 890                 895

Thr Leu Leu Thr Thr Gln Gly Asn Ile Phe Ser Ala Glu Val His Gly
            900                 905                 910

Leu Glu Ser Asp Thr Arg Tyr Phe Phe Lys Met Gly Ala Arg Thr Glu
        915                 920                 925

Val Gly Pro Gly Pro Phe Ser Arg Leu Gln Asp Val Ile Thr Leu Gln
    930                 935                 940

Glu Lys Leu Ser Asp Ser Leu Asp Met His Ser Val Thr Gly Ile Ile
945                 950                 955                 960

Val Gly Val Cys Leu Gly Leu Leu Cys Leu Leu Ala Cys Met Cys Ala
                965                 970                 975

Gly Leu Arg Arg Ser Pro His Arg Glu Ser Leu Pro Gly Leu Ser Ser
            980                 985                 990

Thr Ala Thr Pro Gly Asn Pro Ala Leu Tyr Ser Arg Ala Arg Leu Gly
        995                 1000                1005

Pro Pro Ser Pro Pro Ala Ala His Glu Leu Glu Ser Leu Val His Pro
   1010                1015                1020

His Pro Gln Asp Trp Ser Pro Pro Pro Ser Asp Val Glu Asp Arg Ala
1025                1030                1035                1040

Glu Val His Ser Leu Met Gly Gly Gly Val Ser Glu Gly Arg Ser His
                1045                1050                1055

Ser Lys Arg Lys Ile Ser Trp Ala Gln Pro Ser Gly Leu Ser Trp Ala
            1060                1065                1070

Gly Ser Trp Ala Gly Cys Glu Leu Pro Gln Ala Gly Pro Arg Pro Ala
        1075                1080                1085

Leu Thr Arg Ala Leu Leu Pro Pro Ala Gly Thr Gly Gln Thr Leu Leu
   1090                1095                1100

Leu Gln Ala Leu Val Tyr Asp Ala Ile Lys Gly Asn Gly Arg Lys Lys
1105                1110                1115                1120

Ser Pro Pro Ala Cys Arg Asn Gln Val Glu Ala Glu Val Ile Val His
                1125                1130                1135

Ser Asp Phe Ser Ala Ser Asn Gly Asn Pro Asp Leu His Leu Gln Asp
            1140                1145                1150

Leu Glu Pro Glu Asp Pro Leu Pro Pro Glu Ala Pro Asp Leu Ile Ser
        1155                1160                1165

Gly Val Gly Asp Pro Gly Gln Gly Ala Ala Trp Leu Asp Arg Glu Leu
   1170                1175                1180

Gly Gly Cys Glu Leu Ala Ala Pro Gly Pro Asp Arg Leu Thr Cys Leu
1185                1190                1195                1200

Pro Glu Ala Ala Ser Ala Ser Cys Ser Tyr Pro Asp Leu Gln Pro Gly
                1205                1210                1215

Glu Val Leu Glu Glu Thr Pro Gly Asp Ser Cys Gln Leu Lys Ser Pro
            1220                1225                1230

Cys Pro Leu Gly Ala Ser Pro Gly Leu Pro Arg Ser Pro Val Ser Ser
        1235                1240                1245

Ser Ala
   1250